# EUROPÄISCHE PATENTSCHRIFT

(11) **EP 2 696 837 B1**
(45) Veröffentlichungstag und Bekanntmachung des Hinweises auf die Patenterteilung: **09.09.2015**
(21) Anmeldenummer: 12707531.5
(22) Anmeldetag: 01.03.2012
(51) Int. Cl.: A61J 3/07, B07C 5/34, B65B 1/30, B65B 57/06, G01N 21/95

(54) **VORRICHTUNG ZUR KONTROLLE VON PHARMAZEUTISCHEN PRODUKTEN**
DEVICE FOR CONTROLLING PHARMACEUTICAL PRODUCTS
DISPOSITIF DESTINÉ AU CONTRÔLE DE PRODUITS PHARMACEUTIQUES

(30) Priorität: 13.04.2011 DE 102011007276
(43) Veröffentlichungstag der Anmeldung: 19.02.2014
(73) Patentinhaber: Robert Bosch GmbH, 70442 Stuttgart (DE)
(72) Erfinder: SCHLIPF, Jens, 71691 Freiberg A. N. (DE); RUNFT, Werner, 71364 Winnenden (DE); MAGA, Iulian, 71638 Ludwigsburg (DE); VOGT, Martin, 73614 Schorndorf (DE)
(86) Internationale Anmeldenummer: PCT/EP2012/053545
(87) Internationale Veröffentlichungsnummer: WO 2012/139809

(56) Entgegenhaltungen:
- EP-A1- 2 042 855
- EP-A2- 0 249 791
- DE-B- 1 054 902
- DE-C1- 19 819 395
- US-A- 3 390 769
- US-A- 4 117 935

## Beschreibung

### Stand der Technik

Die Erfindung betrifft eine Vorrichtung zur Kontrolle von pharmazeutischen Produkten nach dem Oberbegriff des Anspruchs 1.

Eine derartige Vorrichtung ist aus der nachveröffentlichten DE 10 2010 038 544 A1 der Anmelderin bekannt. Mittels der bekannten Vorrichtung lässt sich mit Hilfe einer als Röntgenstrahlungsquelle ausgebildeten Strahlungsquelle z.B. das Füllgewicht von in Hartgelatinekapseln befindlichem Füllgut bestimmten. Darüber hinaus ist die Vorrichtung dazu geeignet, z.B. unerwünschte Fremdpartikel oder ähnliches in den Hartgelatinekapseln detektieren zu können. Bei einer in der Fig. 6 der angesprochenen Schrift offenbarten Ausführungsvariante gelangen die pharmazeutischen Produkte aus einem die pharmazeutischen Produkte ungeordnet aufnehmenden Speicherelement in eine schachtförmige Fördereinrichtung, in der die pharmazeutischen Produkte (Hartgelatinekapseln) als Reihe übereinanderstehend angeordnet sind. Um jeweils ein pharmazeutisches Produkt aus der Reihe separieren zu können, ist eine Sperreinrichtung in Form einer Sperrklinke vorgesehen, mit der die jeweils unterste Hartgelatinekapsel der Reihe separiert werden kann. Nur diese Hartgelatinekapsel wird hinsichtlich der oben erwähnten Eigenschaften (Füllgewicht bzw. Fremdkörper) bewertet, indem das Bild der Röntgenstrahlung auf der der Röntgenstrahlungsquelle gegenüberliegenden Seite der Hartgelatinekapsel mittels eines bildaufnehmenden Sensors erfasst und einer Auswerteeinrichtung zugeführt wird. Nachteilig bei der bekannten Vorrichtung ist deren relativ geringe Leistung.

Gattungsgemäße Vorrichtungen zur Kontrolle von pharmazeutischen Produkten sind beispielsweise auch aus der US 4117935 A bzw. EP 2042855 A1 bekannt. Diese offenbaren allerdings nicht, dass jeweils wenigstens zwei Produkte mittels einer Sperreinrichtung aus der Reihe vereinzelt und gleichzeitig im Strahlungsbereich positioniert und untersucht werden.

### Offenbarung der Erfindung

Ausgehend von dem dargestellten Stand der Technik liegt der Erfindung die Aufgabe zugrunde, eine Vorrichtung zur Kontrolle von pharmazeutischen Produkten nach dem Oberbegriff des Anspruchs 1 derart weiterzubilden, dass diese eine gegenüber dem Stand der Technik erhöhte Leistung aufweist. Diese Aufgabe wird bei einer Vorrichtung zur Kontrolle von pharmazeutischen Produkten mit den Merkmalen des Anspruchs 1 erfindungsgemäß dadurch gelöst, dass jeweils wenigstens zwei Produkte mittels der Sperreinrichtung aus der Reihe vereinzelt und gleichzeitig im Strahlungsbereich positioniert und mittels der Sensoreinrichtung erfasst werden. Gegenüber dem Stand der Technik ergibt sich somit zumindest eine Verdoppelung der Leistung.

Vorteilhafte Weiterbildungen der erfindungsgemäßen Vorrichtung zur Kontrolle von pharmazeutischen Produkten sind in den Unteransprüchen angegeben. In den Rahmen fallen sämtliche Kombinationen aus zumindest zwei von in den Ansprüchen, der Beschreibung und/oder den Figuren offenbarten Merkmalen.

In einer konstruktiven Ausgestaltung der Erfindung, bei der die wenigstens zwei Produkte auf einfache Art und Weise von der Reihe separiert werden können, wird vorgeschlagen, dass die Sperreinrichtung zwei, in Wirkverbindung mit den Produkten in dem Förderelement bringbare Sperrelemente aufweist, die voneinander einen Abstand in Förderrichtung der Produkte aufweisen, der der Länge zweier Produkte entspricht.

Um die Vorrichtung für unterschiedliche Formate der Produkte verwenden zu können, wobei sich die Formate in der Länge der jeweiligen Produkte unterscheiden, wird darüber hinaus vorgeschlagen, dass wenigstens ein Sperrelement zur Formatanpassung an die Produkte in Förderrichtung verstellbar angeordnet ist.

Mittels der erfindungsgemäßen Vorrichtung lassen sich Eigenschaften der Produkte ermitteln, die zu einer Gut- bzw. zu einer Schlecht-Bewertung der Produkte führen. Um insbesondere ein Ausscheiden der "Schlecht-Produkte" zu ermöglichen, die ohne manuelle Eingriffe die "Schlecht-Produkte" ausscheidet, wird darüber hinaus in einer besonders bevorzugten Ausgestaltung vorgeschlagen, dass der Fördereinrichtung in Förderrichtung nach der Strahlungsquelle eine Ausscheideeinrichtung nachgeordnet ist.

Um hierbei aus mehreren, zuvor im Bereich der Strahlungsquelle beurteilten Produkten ggf. nur ein einziges Produkt ausscheiden zu können, ist es dabei bevorzugt vorgesehen, dass der Ausscheideeinrichtung im Bereich der Fördereinrichtung zwei in Förderrichtung nacheinander angeordnete weitere Sperrelemente nachgeschaltet sind, die mit einem Weichenelement zusammenwirken.

Die Kontrolle bzw. Bewertung der Eigenschaften der Produkte benötigt in der Praxis einen gewissen Zeitraum. Um trotz des erforderlichen Zeitraums eine möglichst kompakte Vorrichtung ausbilden zu können, ist es in einer weiteren Ausgestaltung der Erfindung vorgesehen, dass dem Weichenelement ein Zwischenspeicherelement vorgeschaltet ist. In diesem Zwischenspeicherelement lassen sich somit die Produkte zwischenspeichern, so dass diese nicht unmittelbar nach dem Verlassen des Bereichs der Strahlungsquelle ausgeschieden werden müssen.

In bevorzugter konstruktiver Ausgestaltung der Erfindung ist das Zwischenspeicherelement als ein um eine Achse schrittweise gedrehter Speicherzylinder ausgebildet, in dem vorzugsweise mehrere, in gleichmäßigen Winkelabständen zueinander angeordnete Aufnahmen für jeweils ein Produkt vorgesehen sind.

Weitere Vorteile, Merkmale und Einzelheiten der Erfindung ergeben sich aus der nachfolgenden Beschreibung bevorzugter Ausführungsbeispiele sowie anhand der Zeichnung.

Diese zeigt in:
- Fig. 1: einen vereinfachten Längsschnitt durch eine erste erfindungsgemäße Vorrichtung zur Kontrolle von pharmazeutischen Produkten mittels einer Röntgenstrahlungsquelle,
- Fig. 2: eine gegenüber der Fig. 1 modifizierte Vorrichtung in perspektivischer Ansicht und
- Fig. 3: einen Teilbereich der Fig. 2 im Bereich eines Zwischenspeicherelements im Längsschnitt.

Gleiche Bauteile bzw. Bauteile mit gleicher Funktion sind in den Figuren mit den gleichen Bezugsziffern versehen.

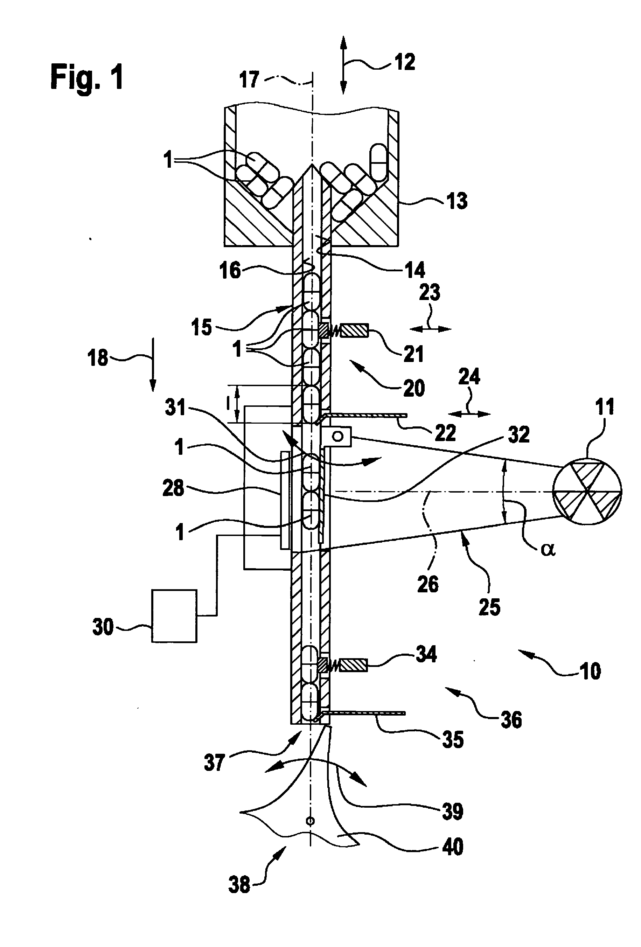

In der Fig. 1 ist eine erste erfindungsgemäße Vorrichtung 10 zur Kontrolle von pharmazeutischen Produkten, insbesondere zur Kontrolle von Hartgelatinekapseln 1, dargestellt. Hierbei werden die Hartgelatinekapseln 1 mittels einer Röntgenstrahlungsquelle 11 durchleuchtet bzw. durchstrahlt, und das Ergebnis der Durchstrahlung wird zur Beurteilung dafür herangezogen, ob es sich bei der Hartgelatinekapsel 1 um eine "Gut-Kapsel" oder um eine "Schlecht-Kapsel" handelt. Mittels der Durchstrahlung der Hartgelatinekapsel 1 lässt sich insbesondere das Füllgewicht des in der Hartgelatinekapsel 1 befindlichen Füllguts oder das Vorhandensein von Fremdkörpern detektieren bzw. feststellen. Bezüglich Einzelheiten einer derartigen Detektion bzw. Auswertung wird auf die nachveröffentlichte DE 10 2010 038 544 A1 der Anmelderin verwiesen, die insofern Bestandteil dieser Anmeldung sein soll.

Ergänzend wird erwähnt, dass anstelle von Hartgelatinekapseln 1 auch andere pharmazeutische Produkte, wie HPMC-Kapseln, Oblong-Tabletten o.ä. mittels der Vorrichtung 10 verarbeitet werden können.

Die Vorrichtung 10 weist einen in Richtung des Doppelpfeils 12 auf- und abbeweglichen Vorrats- bzw. Speicherbehälter 13 für die Hartgelatinekapseln 1 auf, in dem eine große Anzahl von Hartgelatinekapseln 1 ungeordnet aufgenommen ist. Im Bodenbereich des Speicherbehälters 13 ist eine Bohrung 14 ausgebildet, die von einem rohr- oder schachtförmigen Förderelement 15 durchsetzt ist. Das Förderelement 15 weist wenigstens eine, bevorzugt jedoch mehrere, senkrecht zur Zeichenebene der Fig. 1 angeordnete Durchgangsbohrungen 16 auf, wobei der Durchmesser der Durchgangsbohrung 16 dem Durchmesser der länglichen, eine Länge I aufweisenden Hartgelatinekapseln 1 derart angepasst ist, dass in der Durchgangsbohrung 16 die Hartgelatinekapseln 1 als Reihe gefördert werden können. Die Längsachse 17 der Durchgangsbohrung 16 ist vorzugsweise vertikal angeordnet.

Mittels des Förderelements 15 lassen sich die Hartgelatinekapseln 1 in Richtung des Pfeils 18 fördern, wobei sich diese allein durch ihre Gewichtskraft in Richtung des Pfeils 18 bewegen. Unterhalb des Speicherbehälters 13 ist eine Sperreinrichtung 20 zum Vereinzeln jeweils wenigstens zweier, übereinander stehender Hartgelatinekapseln 1 angeordnet. Die Sperreinrichtung 20 umfasst zwei, in Förderrichtung der Hartgelatinekapseln 1 nacheinander angeordnete Sperrelemente 21, 22, die die Wand des Förderelements 15 durchsetzen, und die mittels nicht dargestellter Antriebe in Richtung der Doppelpfeile 23, 24 quer zur Längsachse 17 des Förderelements 15 bewegbar sind. In der freigebenden Stellung der Sperrelemente 21, 22 sind sie dabei derart positioniert, dass im Bereich der Sperrelemente 21, 22 befindliche Hartgelatinekapseln 1 sich ungehindert sich in Richtung des Pfeils 18 bewegen können. Demgegenüber werden die Hartgelatinekapseln 1 in der Sperrstellung der Sperrelemente 21, 22 von dem jeweiligen Sperrelement 21, 22 gehalten, indem das jeweilige Sperrelement 21, 22 in Kontakt mit der betreffenden Hartgelatinekapsel 1 gerät und diese dabei gegen die dem Sperrelement 21, 22 gegenüberliegende Wand des Förderelements 15 drückt.

Zur Formatanpassung kann es vorgesehen sein, dass wenigstens eines der Sperrelemente 21, 22 in der Richtung der Längsachse 17 verstellbar angeordnet ist, so dass insbesondere bei einer Formateinstellung, bei der die Länge I der Hartgelatinekapseln 1 gegenüber zuvor verwendeten Hartgelatinekapseln 1 größer ist der Abstand zwischen den beiden Sperrelementen 21, 22 vergrößert wird.

Unterhalb der Sperreinrichtung 20 befindet sich seitlich die Röntgenstrahlungsquelle 11, die einen Strahlungskegel 25 mit beispielsweise einem Öffnungswinkel a von 30° erzeugt. Die Mittelachse 26 des Strahlungskegels 25 verläuft bevorzugt, jedoch nicht zwingend, senkrecht zur Längsachse 17 der Durchgangsbohrung 16. Das Förderelement 15 besteht im Bereich des Strahlungskegels 25 aus einem für die Röntgenstrahlung durchlässigen Material, beispielsweise aus Kunststoff, Glas oder ähnlichem. Auf der der Röntgenstrahlungsquelle 11 gegenüberliegenden Seite der Hartgelatinekapseln 1 weist das Förderelement 15 entweder eine entsprechende Aussparung für wenigstens ein Sensorelement 28 auf, oder aber das Förderelement 15 besteht in diesem Bereich ebenfalls aus einem für die Röntgenstrahlung durchlässigen Material.

Das Sensorelement 28 ist als bildaufnehmendes Sensorelement 28 ausgebildet, das ein Bild der Röntgenstrahlung der durchstrahlten Hartgelatinekapseln 1 aufnimmt und einer Auswerteeinrichtung 30 zuführt. Die Auswerteeinrichtung 30 überprüft mittels eines Algorithmus die im Bereich des Sensorelements 28 durchleuchteten Hartgelatinekapseln 1 auf die zu überprüfende Eigenschaft, beispielsweise auf das Füllgewicht oder das Vorhandensein von Verunreinigungen, Fremdpartikeln oder ähnlichem.

Im Bereich des Sensorelements 28 weist das Förderelement 15 ein in Richtung des Doppelpfeils 31 schwenkbares Klemmelement 32 in Form einer Klemmplatte auf, das zumindest in Wirkverbindung mit der unteren der wenigstens zwei Hartgelatinekapseln 1 in seiner Sperrstellung bringbar ist, und dabei die zumindest untere der Hartgelatinekapseln 1 entweder gegen die Wand des Förderelements 15, oder aber gegen das Sensorelement 28 drückt und somit diejenigen Hartgelatinekapseln 1 im Bereich des Sensorelements 28 festhält, die während eines Prüfvorgangs durchstrahlt werden.

Unterhalb der Röntgenstrahlungsquelle 11 bzw. des Sensorelements 28 sind beabstandet zum Sensorelement 28 zwei weitere Sperrelemente 34, 35 einer weiteren Sperreinrichtung 36 angeordnet. Die weiteren Sperrelemente 34, 35 sind vorzugsweise identisch zu den Sperrelementen 21, 22 ausgebildet, so dass diesbezüglich auf die obige Erläuterung verwiesen wird. Mittels der weiteren Sperreinrichtung 36 lässt sich jede der im Bereich der Sperreinrichtung 36 befindlichen Hartgelatinekapseln 1 vereinzeln. Insbesondere dient die Sperreinrichtung 36 dazu, "Schlecht-Kapseln" von "Gut-Kapseln" zu trennen. Hierzu dient eine dem Ausgang 37 des Förderelements 15 nachgeschaltete Ausscheideeinrichtung 38 in Form einer in Richtung des Doppelpfeils 39 schwenkbaren Ausscheideklappe 40.

Die soweit beschriebene Vorrichtung 10 arbeitet wie folgt: Durch eine Auf- und Abbewegung des Speicherbehälters 13 werden in dem Speicherbehälter 13 befindliche Hartgelatinekapseln 1 in die Durchgangsbohrung 16 bzw. die Durchgangsbohrungen 16 des Förderelements 15 gefördert, so dass diese oberhalb der Sperreinrichtung 20 als Reihe anstehen. Mittels der Sperreinrichtung 20 werden jeweils wenigstens zwei übereinander in Längsrichtung stehende Hartgelatinekapseln 1 aus der Reihe vereinzelt und in den Bereich des Sensorelements 28 gefördert, wobei die Klemmeinrichtung 32 sich in ihrer Klemmposition befindet. Wichtig dabei ist, dass die zwei Hartgelatinekapseln 1 dabei von den anderen, oberhalb befindlichen Hartgelatinekapseln 1 mit Ausnahme des Bereichs, an denen die beiden Hartgelatinekapseln 1 sich berühren, freigestellt werden. Sobald die wenigstens zwei Hartgelatinekapseln 1 sich im Bereich des Strahlungskegels 25 der Röntgenstrahlungsquelle 11 in ihrer Stillstandsposition befinden, wird mittels des Sensorelements 28 ein Bild der durchstrahlten Hartgelatinekapseln 1 aufgenommen und mittels der Auswerteeinrichtung 30 analysiert bzw. bewertet. Anschließend wird die Klemmeinrichtung 32 geschwenkt, so dass die Hartgelatinekapseln 1 aus dem Bereich des Sensorelements 28 freigegeben werden und zunächst im Bereich der weiteren Sperreinrichtung 36 festgehalten werden. Sofort nach dem Freigeben der beiden Hartgelatinekapseln 1 aus dem Bereich des Sensorelements 28 wird das Klemmelement 32 wieder in seine Sperrposition verbracht, so dass die nächsten beiden Hartgelatinekapseln 1 untersucht werden können. Sobald das Ergebnis des von dem Sensorelement 28 erfasste Bildes durch die Auswerteeinrichtung 30 feststeht, wird die zweite Sperreinrichtung 36 derart angesteuert, dass je nach Position der Ausscheideklappe 40 die untersuchten Hartgelatinekapseln 1 entweder weiterbearbeitet werden, oder aber ausgeschieden werden.

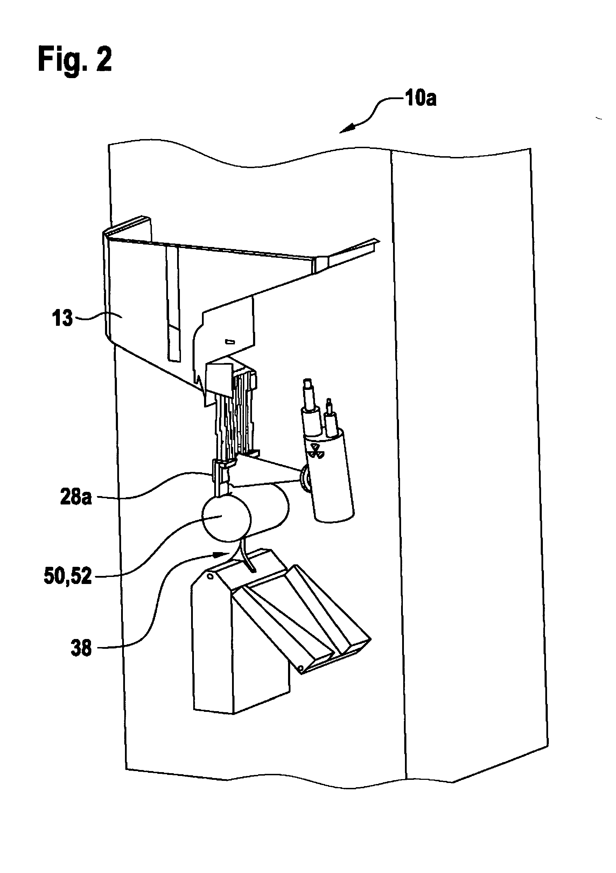

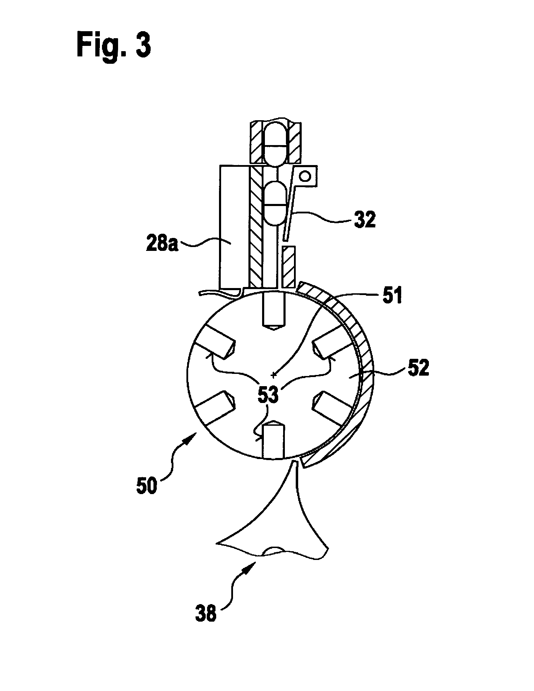

In den Fig. 2 und 3 ist eine modifizierte Vorrichtung 10a dargestellt. Wesentlich hierbei ist, dass sich bei der Vorrichtung 10a an den Bereich des Sensorelements 28a ein Zwischenspeicherelement 50 anschließt. Das Zwischenspeicherelement 50 ersetzt die zweite Sperreinrichtung 36 bei der Vorrichtung 10. Der Zwischenspeicher 50 ist in Form eines in einer horizontalen Drehachse 51 schrittweise gedrehten Speicherzylinders 52 ausgebildet. Am Umfang des Speicherzylinders 52 sind vorzugsweise in gleichmäßigen Winkelabständen zueinander angeordnete Aufnahmen 53 ausgebildet, die in Form einer Bohrung jeweils eine einzige Hartgelatinekapsel 1 aufnehmen. In der Übernahmeposition des Zwischenspeichers 50 zur Übernahme der jeweils unteren Hartgelatinekapsel 1 aus dem Bereich des Sensorelements 28a sowie in der Ausscheideposition der Hartgelatinekapsel 1 aus der Aufnahme 53 des Zwischenspeichers 50 sind die betreffenden Aufnahmen 53 bevorzugt vertikal ausgerichtet. Der Zwischenspeicher 50 ermöglicht somit insbesondere beim Vorhandensein einer Vielzahl von Aufnahmen 53 die Zwischenspeicherung von bereits durchstrahlten Hartgelatinekapseln 1, um während der Zwischenspeicherung genügend Zeit für die Auswerteeinrichtung 30 bereitzustellen, damit festgestellt werden kann, ob die Hartgelatinekapsel 1 die gewünschten Eigenschaften erfüllt oder nicht.

Die soweit beschriebenen Vorrichtungen 10, 10a können in vielfältiger Art und Weise abgewandelt bzw. modifiziert werden, ohne vom Erfindungsgedanken abzuweichen. Dieser besteht darin, jeweils wenigstens zwei, übereinander angeordnete Hartgelatinekapseln 1 in den Bereich der Röntgenstrahlungsquelle 11 zu fördern und gleichzeitig zu untersuchen.

## Patentansprüche

1. Vorrichtung (10; 10a) zur Kontrolle von pharmazeutischen Produkten (1), insbesondere von Hartgelatinekapseln, mittels einer vorzugsweise als Röntgenstrahlungsquelle ausgebildeten Strahlungsquelle (11), mit einer die Produkte (1) ungeordnet aufnehmenden Speichereinrichtung (13), aus der die Produkte (1) in ein Förderelement (15) übergeben werden, in der die Produkte (1) in Form wenigstens einer Reihe angeordnet sind, wobei die Produkte (1) taktweise in den Strahlungsbereich (25) der Strahlungsquelle (11) gefördert werden, die die Produkte (1) vorzugsweise senkrecht zu deren Längsrichtung durchstrahlt, wobei der Strahlungsquelle (11) in Förderrichtung (18) der Produkte (1) eine erste Sperreinrichtung (20) zum Vereinzeln von Produkten (1) der Reihe vorgeschaltet ist, und wobei wenigstens ein, mit einer Auswerteeinrichtung (30) gekoppeltes Sensorelement (28; 28a) vorgesehen ist, das die Strahlung der durchstrahlten Produkte (1) erfasst,
**dadurch gekennzeichnet,**
**dass** jeweils wenigstens zwei Produkte (1) mittels der ersten Sperreinrichtung (20) aus der Reihe vereinzelt und gleichzeitig im Strahlungsbereich (25) positioniert und mittels des wenigstens einen Sensorelements (28; 28a) erfasst werden, und dass im Strahlungsbereich (25) ein Klemmelement (32) zum Positionieren der wenigstens zwei Produkte (1) vor dem Sensorelement (28; 28a) vorgesehen ist.

2. Vorrichtung nach Anspruch 1,
**dadurch gekennzeichnet,**
**dass** die erste Sperreinrichtung (20) zwei, in Wirkverbindung mit den Produkten (1) in dem Förderelement (15) bringbare Sperrelemente (21, 22) aufweist, die voneinander einen Abstand in Förderrichtung (18) der Produkte (1) aufweisen, der der Länge (I) wenigstens zweier Produkte (1) entspricht.

3. Vorrichtung nach Anspruch 2,
**dadurch gekennzeichnet,**
**dass** wenigstens ein der zwei Sperrelemente (21, 22) zur Formatanpassung an die Produkte (1) in Förderrichtung (18) verstellbar angeordnet ist.

4. Vorrichtung nach einem der Ansprüche 1 bis 3,
**dadurch gekennzeichnet,**
**dass** dem Förderelement (15) in Förderrichtung (18) nach der Strahlungsquelle (11) eine Ausscheideeinrichtung (38) nachgeordnet ist.

5. Vorrichtung nach Anspruch 4,
**dadurch gekennzeichnet,**
**dass** der Ausscheideeinrichtung (38) im Bereich des Förderelements (15) zwei in Förderrichtung (18) nacheinander angeordnete weitere Sperrelemente (34, 35) vorgeschaltet sind, die mit einem nachgeschalteten Weichenelement (40) der Ausscheideeinrichtung (38) zusammenwirken.

6. Vorrichtung nach Anspruch 4,
**dadurch gekennzeichnet,**
**dass** dem Weichenelement (40) ein Zwischenspeicherelement (50) vorgeschaltet ist.

7. Vorrichtung nach Anspruch 6,
**dadurch gekennzeichnet,**
**dass** das Zwischenspeicherelement (50) ein um eine Drehachse (51) schrittweise gedrehter Speicherzylinder (52) ist, in dem vorzugsweise mehrere, in gleichmäßigen Winkelabständen zueinander angeordnete Aufnahmen (52) für jeweils ein Produkt (1) vorgesehen sind.

8. Vorrichtung nach Anspruch 7,
**dadurch gekennzeichnet,**
**dass** die Drehachse (51) des Speicherzylinders (52) horizontal ausgerichtet ist, und dass die Aufnahmen (52) in einer Übernahme- und einer Ausscheideposition der Produkte (1) zumindest in etwa vertikal ausgerichtet sind.

9. Vorrichtung nach Anspruch 1,
**dadurch gekennzeichnet,**
**dass** das Klemmelement (32) als schwenkbare Klemmplatte ausgebildet ist.

## Claims

1. Device (10; 10a) for monitoring pharmaceutical products (1), in particular hard gelatine capsules, by means of a radiation source (11) preferably embodied as an X-ray radiation source, said device comprising a storage apparatus (13) receiving the products (1) in a disordered fashion, from where the products (1) are transferred into a conveying element (15), in which the products (1) are arranged in the form of at least one row, wherein the products (1) are conveyed in a clocked manner to the radiation region (25) of the radiation source (11), which preferably irradiates the products (1) perpendicular to the longitudinal direction thereof, wherein a first blocking apparatus (20) is disposed upstream of the radiation source (11) in the conveying direction (18) of the products (1) for separating products (1) of the row and wherein provision is made for at least one sensor element (28; 28a), which is coupled to an evaluation apparatus (30) and registers the radiation of the irradiated products (1),
**characterized**
**in that** at least two products (1) are separated from the row by means of the first blocking apparatus (20) in each case and simultaneously positioned in the radiation region (25) and registered by means of the at least one sensor element (28; 28a), and in that provision is made in the radiation region (25) for a clamping element (32) for positioning the at least two products (1) in front of the sensor element (28; 28a).

2. Device according to Claim 1,
**characterized**
**in that** the first blocking apparatus (20) has two blocking elements (21, 22) which can establish a functional connection with the products (1) in the conveying element (15), said blocking elements being at a distance from one another corresponding to the length (1) of at least two products (1) in the conveying direction (18) of the products (1).

3. Device according to Claim 2,
**characterized**
**in that** at least one of the two blocking elements (21, 22) is arranged in a manner adjustable in the conveying direction (18) for adapting the format to the products (1).

4. Device according to one of Claims 1 to 3,
**characterized**
**in that** a rejection apparatus (38) is arranged downstream of the conveying element (15), after the radiation source (11), in the conveying direction (18).

5. Device according to Claim 4,
**characterized**
**in that** two further blocking elements (34, 35) arranged in succession in the conveying direction (18) are disposed upstream of the rejection apparatus (38) in the region of the conveying element (15), which blocking elements interact with a separator element (40) of the rejection apparatus (38) disposed downstream thereof.

6. Device according to Claim 4,
**characterized**
**in that** a buffer storage element (50) is disposed upstream of the separator element (40).

7. Device according to Claim 6,
**characterized**
**in that** the buffer storage element (50) is a storage cylinder (52) rotated incrementally about an axis of rotation (51), in which storage cylinder provision is preferably made for a plurality of receptacles (52) for respectively one product (1) arranged at regular angle distances in relation to one another.

8. Device according to Claim 7,
**characterized**
**in that** the axis of rotation (51) of the storage cylinder (52) is aligned horizontally and in that the receptacles (52) are aligned at least approximately vertically in a reception and discharge position of the products (1).

9. Device according to Claim 1,
**characterized**
**in that** the clamping element (32) is embodied as swivelable clamping plate.

## Revendications

1. Dispositif (10 ; 10a) pour le contrôle de produits pharmaceutiques (1), en particulier de capsules de gélatine dure, au moyen d'une source de rayonnement (11) réalisée de préférence en tant que source de rayonnement X, comprenant un dispositif de stockage (13) recevant les produits (1) de manière désordonnée, à partir duquel les produits (1) sont transférés dans un élément de transport (15) dans lequel les produits (1) sont disposés sous la forme d'au moins une rangée, les produits (1) étant transportés de manière cadencée dans la région de rayonnement (25) de la source de rayonnement (11) qui irradie les produits (1) de préférence perpendiculairement à leur direction longitudinale, un premier dispositif de blocage (20) pour séparer des produits (1) de la rangée étant placé en amont de la source de rayonnement (11) dans le sens de transport (18) des produits (1), et au moins un élément de capteur (28 ; 28a) accouplé à un dispositif d'évaluation (30) étant prévu, lequel élément de capteur détecte le rayonnement des produits irradiés (1),
**caractérisé en ce**
**qu'**à chaque fois au moins deux produits (1) sont séparés à partir de la rangée au moyen du premier dispositif de blocage (20) et sont positionnés simultanément dans la région de rayonnement (25) et sont détectés au moyen de l'au moins un élément de capteur (28 ; 28a), et en ce qu'un élément de serrage (32) est prévu dans la région de rayonnement (25) pour le positionnement des au moins deux produits (1) devant l'élément de capteur (28 ; 28a).

2. Dispositif selon la revendication 1,
**caractérisé en ce que**
le premier dispositif de blocage (20) comprend deux éléments de blocage (21, 22) pouvant être amenés en liaison fonctionnelle avec des produits (1) dans l'élément de transport (15), lesquels éléments de blocage sont espacés l'un de l'autre d'une certaine distance dans le sens de transport (18) des produits (1), laquelle distance correspond à la longueur (1) d'au moins deux produits (1).

3. Dispositif selon la revendication 2,
**caractérisé en ce**
**qu'**au moins l'un des deux éléments de blocage (21, 22) est disposé de manière déplaçable dans le sens de transport (18) pour l'adaptation au format des produits (1).

4. Dispositif selon l'une quelconque des revendications 1 à 3,
**caractérisé en ce**
**qu'**un dispositif de rejet (38) est placé en aval de l'élément de transport (15) dans le sens de transport (18), après la source de rayonnement (11).

5. Dispositif selon la revendication 4,
**caractérisé en ce que**
deux éléments de blocage supplémentaires (34, 35) disposés l'un après l'autre dans le sens de transport (18) sont placés en amont du dispositif de rejet (38) dans la région de l'élément de transport (15), lesquels éléments de blocage coopèrent avec un élément d'aiguillage (40), placé en aval, du dispositif de rejet (38).

6. Dispositif selon la revendication 4,
**caractérisé en ce**
**qu'**un élément de stockage temporaire (50) est placé en amont de l'élément d'aiguillage (40).

7. Dispositif selon la revendication 6,
**caractérisé en ce que**
l'élément de stockage temporaire (50) est un cylindre de stockage (52) tourné graduellement autour d'un axe de rotation (51), dans lequel cylindre de stockage sont prévus de préférence plusieurs logements (52) pour un produit respectif (1), lesquels logements sont disposés à des écarts angulaires réguliers les uns par rapport aux autres.

8. Dispositif selon la revendication 7,
**caractérisé en ce que**
l'axe de rotation (51) du cylindre de stockage (52) est orienté horizontalement, et **en ce que** les logements (52) sont orientés au moins approximativement verticalement dans une position de reprise et une position de rejet des produits (1).

9. Dispositif selon la revendication 1,
**caractérisé en ce que**
l'élément de serrage (32) est réalisé sous forme de plaque de serrage pivotante.
